# Europäisches Patentamt
# European Patent Office
# Office européen des brevets

(19)

(11) Veröffentlichungsnummer: **0 151 291**
**B1**

(12) ## EUROPÄISCHE PATENTSCHRIFT

(45) Veröffentlichungstag der Patentschrift:
03.09.86

(51) Int. Cl.⁴: **C 07 C 85/12, B 01 J 23/74**

(21) Anmeldenummer: **84115715.9**

(22) Anmeldetag: **18.12.84**

(54) Geformte Eisenkatalysatormasse, deren Herstellung und Verwendung.

(30) Priorität: **27.01.84 DE 3402734**

(43) Veröffentlichungstag der Anmeldung:
**14.08.85 Patentblatt 85/33**

(45) Bekanntmachung des Hinweises auf die Patenterteilung:
**03.09.86 Patentblatt 86/36**

(84) Benannte Vertragsstaaten:
**BE CH DE FR GB IT LI NL**

(56) Entgegenhaltungen:
**EP-A-0 101 584**
**EP-A-0 103 723**
**GB-A-496 880**
**US-A-3 846 286**
**US-A-4 410 448**

**CHEMICAL ABSTRACTS, Band 84, Nr. 10, 8. März 1976, Seite 431, Nr. 65753b, Columbus, Ohio, US;**

(73) Patentinhaber: **BASF Aktiengesellschaft, Carl-Bosch- Strasse 38, D-6700 Ludwigshafen (DE)**

(72) Erfinder: **Frank, Gerhard, Dr., Heidelberger Strasse 11, D-6945 Hirschberg (DE)**
Erfinder: **Neubauer, Gerald, Dr., Mozartstrasse 24, D-6940 Weinheim (DE)**

**Beschreibung**

Gegenstand der Erfindung ist eine geformte Eisenkatalysatormasse, enthaltend metallische Eisenteilchen und Gleitmittel sowie deren Herstellung und Verwendung.

Bei der Herstellung von Aminen durch Hydrieren von Nitrilen, z.B. Hexamethylendiamin aus Adiponitril, werden Kobalt enthaltende Katalysatoren wegen ihrer hohen Selektivität bevorzugt verwendet. Solche Verfahren sind beispielsweise aus den DE-PSen 1 072 972 und 1 259 899 bekannt. Die Lebensdauer der verwendeten Kobaltkatalysatoren entspricht jedoch nicht mehr den technischen Anforderungen. Es hat sich nämlich herausgestellt, daß solche Kobaltkatalysatoren beim Gebrauch zunehmend zerfallen. Darüber hinaus weisen Befunde darauf hin, daß Stäube metallischen Kobalts und seiner schwerlöslichen Verbindungen aus arbeitshygienischen Gründen vermieden werden sollten.

Es wurden auch schon Eisen enthaltende Katalysatoren für die Hydrierung von Nitrilen zu Aminen verwendet. Bei der Verwendung von Eisenkatalysatoren müssen jedoch höhere Temperaturen angewandt werden. Dies führt in verstärktem Umfang zur Bildung von Nebenprodukten wie Azacycloheptan und schwer vom Hexamethylendiamin abtrennbaren Diaminen, wie 2-Aminomethyl-cyclopentylamin und 1,2-Diaminocyclohexan sowie zur Bildung von Bishexamethylentriamin und Oligomeren. Aus der DE-OS 2 429 293 ist bekannt, daß man durch Schmelzen von Magnetit und Reduktion mit Wasserstoff einen Katalysator erhält, der bei hot spot Temperaturen von 150 bis 170° C eine Selektivität von 98 bis 99 % an Hexamethylendiamin erzielt. Die Gehalte an 1,2-Diaminocyclohexan liegen jedoch bei 0,2 Gew.%. Die bekannten Katalysatoren sind deshalb noch verbesserungsbedürftig.

Es war deshalb die technische Aufgabe gestellt, Katalysatoren zur Verfügung zu stellen, die bei der Hydrierung von Nitrilen zu Aminen eine lange Lebensdauer haben und nicht zum Zerfallen neigen und darüber hinaus niedrigere Hydriertemperaturen erlauben, wenig Nebenprodukte erzeugen und eine hohe Selektivität aufweisen.

Diese Aufgabe wird gelöst durch geformte Eisenkatalysatormassen enthaltend metallischen Eisenteilchen, die aus Eisenoxidteilchen durch Kontakt mit Wasserstoff bei einer Temperatur von ≦ 500° C erhalten worden sind, und ein Gleitmittel, und die gekennzeichnet sind durch eine Druckhärte von 850 bis 1500 kp/cm².

Gegenstand der Erfindung ist ferner ein Verfahren zur Herstellung von geformten Eisenkatalysatormassen enthaltend, metallische Eisenteilchen und ein Gleitmittel, durch

a) Reduktion von Eisenoxidteilchen mit Wasserstoff bei einer Temperatur von 250 bis 500° C zu metallischen Eisenteilchen

b) Passivieren der Eisenteilchen durch Behandeln mit einem molekularen Sauerstoff enthaltenden Inertgas

c) Pressen der passivierten Eisenteilchen mit Gleitmitteln zu Formkörpern

d) Aktivieren der Formkörper durch Behandeln mit Wasserstoff bei einer Temperatur von 250 bis 500° C

dadurch gekennzeichnet, daß man die aktivierte geformte Eisenkatalysatormasse mindestens noch einmal durch Behandeln mit molekularem Sauerstoff enthaltendem Inertgas passiviert und anschließende Behandlung mit Wasserstoff bei einer Temperatur von 250 bis 500° C aktiviert.

Ferner ist ein Gegenstand der Erfindung die Verwendung der geformten Eisenkatalysatormassen zur Hydrierung von organischen Nitrilen zu den entsprechenden Aminen.

Die neuen Eisenkatalysatormassen haben den Vorteil, daß sie eine lange Lebensdauer haben und sich durch ihre Härte auch nach längerem Gebrauch durch überlegene mechanische Eigenschaften auszeichnen. Die neuen Katalysatoren haben ferner den Vorteil, daß sie bei niedrigeren Temperaturen eine hohe Selektivität haben. Darüber hinaus zeichnen sich die neuen Katalysatoren dadurch aus, daß weniger Nebenprodukte anfallen, die schwer von den Wertprodukten abtrennbar sind.

Die erfindungsgemäße Katalysatormasse enthält metallische Eisenteilchen, die aus Eisenoxidteilchen mit einer mittleren Teilchengröße von vorzugsweise 0,1 bis 2μm, insbesondere von 0,2 bis 1,2μm durch Kontakt mit Wasserstoff bei einer Temperatur von ≦ 500° C erhalten worden sind. Vorzugsweise weisen die metallischen Eisenteilchen einen Reduktionsgrad größer 95 % auf. Unter Reduktionsgrad ist der Anteil des verfügbaren Eisens in Prozent zu verstehen, der in metallischer Form vorliegt.

Bevorzugt werden anisometrische, z.B. nadelförmige gamma-Eisenoxide, insbesondere gamma-Eisen(III)oxid und gamma-Eisen(III)oxidhydrat angewandt. Gamma-Eisen(III)oxidhydrat, das unter dem Namen Lepidokrokit bekannt ist, wird bevorzugt verwendet. Es ist z.B. nach dem in der DE-AS 1 061 760 beschriebenen Verfahren erhältlich. Die anisometrischen Eisenoxide haben in der Regel eine Teilchenlänge von 0,1 bis 2μm bei einem Längen- zu Dickenverhältnis von 5: 1 bis 40: 1 und eine spezifische Oberfläche nach BET von 25 bis 80 m². In gleicher Weise lassen sich auch die getemperten Produkte der genannten III-Eisenoxide verwenden, wobei die Temperung zweckmäßigerweise bei 250 bis 700° C erfolgt. Vorteilhaft haben die verwendeten Eisenoxide einen Alkaligehalt von kleiner 0,1 Gew.%, berechnet als $Na_2O$.

Die erfindungsgemäße Eisenkatalysatormasse enthält ferner Gleitmittel, z.B. anorganische Stoffe mit Gitterstruktur, wie Talkum oder Graphit. Vorteilhaft enthalten die Katalysatoren 1 bis 5 Gew.% Gleitmittel, bezogen auf die gesamte Katalysatormasse aus Eisenteilchen und Gleitmittel. Besonders bewährt hat sich Graphit als Gleitmittel. Die erfindungsgemäße Katalysatormasse besteht somit im wesentlichen aus metallischen Eisenteilchen, geringe Mengen Eisenoxid entsprechend dem Reduktionsgrad und einem Gleitmittel.

Die erfindungsgemäße Eisenkatalysatormasse ist geformt, z.B. als Kugeln, Tabletten oder Strängen und weist eine Druckhärte von 850 bis 1500, insbesondere 900 bis 1300 kp/cm$^2$ auf.

Die erfindungsgemäßen Katalysatormassen stellt man vorteilhaft her, indem man Eisenoxidteilchen z.B. gamma-Eisen(III)oxide, insbesondere gamma-Eisen(III)oxidhydrat (Lepidokrokit) verwendet. In gleicher Weise lassen sich auch die getemperten Produkte der genannten Eisen(III)oxide verwenden, wobei die Temperung zweckmäßigerweise bei 250 bis 700°C erfolgt. Gamma-Eisen(III)oxidhydrat erhält man beispielsweise aus wäßrigen Lösungen von Eisensalzen mit Natronlauge nach einem Verfahren, wie es in der DE-AS 1 061 760 beschrieben wird. Vorteilhaft wäscht man die gamma-Eisenoxidhydratpartikel bis der Alkaligehalt kleiner 0,1 Gew.%, berechnet als Na$_2$O beträgt.

Die nadelförmigen Eisen(III)oxidteilchen werden mittels Wasserstoff, z.B. in der Wirbelschicht im Drehrohrofen oder vorzugsweise in einem gerührten Festbett bei einer Temperatur von 260 bis 500°C, insbesondere 300 bis 450°C, z.B. innerhalb von 3 bis 36 Stunden reduziert. Vorteilhaft wendet man trockenen Wasserstoffstrom an, wobei man eine relativ große Wasserströmungsgeschwindigkeit einhält. Es hat sich bewährt, wenn man mindestens einen 60-fachen Wasserstoffüberschuß anwendet. Vorzugsweise reduziert man solange, bis der Reduktionsgrad größer 95 % beträgt. So erhaltene, im wesentlichen aus Eisen bestehende Metallteilchen besitzen noch weitgehend die von den Ausgangsstoffen herrührende Gestalt und sind trotz der vorangegangenen Umwandlungsreaktion einheitlich.

Anschließend werden die metallischen Eisenteilchen passiviert. Darunter versteht man das Umhüllen der Metallteilchen mit einer Oxidschicht durch kontrollierte Oxidation, um die durch die große freie Oberfläche der kleinen Teilchen bedingte Pyrophorität zu beseitigen. Dies wird durch Überleiten eines molekularen Sauerstoff enthaltenden Inertgases, z.B. Luft/Stickstoffgemisches unter genauer Einhaltung einer vorzugsweise 100°C, insbesondere 80°C nicht übersteigenden Temperatur über das Metallpulver erreicht. Nach der Stabilisierung liegt der Reduktionsgrad vorteilhaft nicht unter 80 %, vorzugsweise nicht unter 90 %. Die stabilisierten Eisenteilchen haben in der Regel eine Oberfläche nach BET von 4 bis 25 m$^2$/g, vorzugsweise 8 bis 12 m$^2$/g sowie Partikellängen von 0,05 bis 2μm.

Die so passivierten Eisenteilchen werden mit einem inerten Gleitmittel, vorzugsweise Graphit, vermischt. Dabei wendet man vorteilhaft 2 bis 5 Gew.% Gleitmittel, bezogen auf die Summe aus Eisenteilchen und Gleitmittel an. Das Gemisch aus passivierten Eisenteilchen und Gleitmittel wird, vorteilhaft unter Stickstoffabdeckung, zu Formkörpern verarbeitet, z.B. zu Tabletten verpreßt. Die Druckhärte der Formkörper soll etwa 300 kp/cm$^2$ betragen.

Die so erhaltenen Formkörper werden durch Behandeln mit Wasserstoff in relativ großem Überschuß bei einer Temperatur von 250 bis 500°C, insbesondere 300 bis 360°C bei Atmosphärendruck oder unter erhöhtem Druck, z.B. 100 bis 150 bar, aktiviert. Hierbei soll ein Reduktionsgrad von vorteilhaft größer 95 % erreicht werden. Durch die Aktivierung steigt die Druckhärte der Formkörper z.B. von 300 auf 600 bis 800 kp/cm$^2$.

Ein wesentliches Merkmal der Erfindung ist es, daß die so erhaltenen aktivierten, geformten Eisenkatalysatormassen (Formkörper) mindestens noch einmal durch Behandeln mit molekularem Sauerstoff enthaltendem Inertgas passiviert und durch anschließende Behandlung mit Wasserstoff bei einer Temperatur von 250 bis 500°C aktiviert werden. Die Bedingungen für die Passivierung und Aktivierung entsprechen den vorgenannten Bedingungen. Diese Passivierung und Aktivierung wird vorteilhaft 1 bis 5 mal, z.B. 2 bis 4 mal, wiederholt. Mit jedem Aktivierungsvorgang steigt die Druckhärte der Formkörper und erreicht einen Wert von 850 bis 1500, insbesondere von 900 bis 1300 kp/cm$^2$.

Die erfindungsgemäßen geformten Eisenkatalysatoren weisen eine hohe Aktivität auf, die es erlaubt, bei Hydriertemperaturen unterhalb 120°C zu arbeiten, während gemäß dem Stand der Technik bei hot-spot Temperaturen bis zu 150°C und darüber gearbeitet werden muß. Auffallend ist dabei seine geringe Neigung zur Bildung von unerwünschten cyclischen Nebenprodukten. So liegen bei der Herstellung von Hexamethylendiamin aus Adipodinitril die Konzentrationen für 1,2-Diaminocyclohexan und Azacycloheptan deutlich unter 0,2 % und für 2-Aminomethylcyclopentylamin unter 0,002 %. Der erfindungsgemäße Katalysator hat durch seine Härte eine ausgezeichnete mechanische Stabilität. Dies wird dadurch erreicht, daß man seine Verformung zu Formkörpern nicht auf der Stufe der Eisen(III)oxide vornimmt, sondern erst nach deren Reduktion zu Eisenmetallteilchen und nachfolgender Passivierung. Eine überragende Härte wird dann erzielt, indem man die geformten Eisenkatalysatormassen abwechselnd passiviert und aktiviert, bis eine Härte von 850 bis 1500 kp/cm$^2$ erreicht ist. Geht man von Formlingen aus, die aus Eisen(III)oxid und Gleitmitteln hergestellt sind und anschließend reduziert worden sind, so geht die Druckhärte der Formlinge z.B. von 300 kp/cm$^2$ auf 25 kp/cm$^2$ nach Erreichen eines Reduktionsgrades von 95 % zurück. Die Katalysatorstandzeiten liegen hierbei unter 100 Tagen.

Die erfindungsgemäßen Katalysatoren eignen sich vorteilhaft zur Hydrierung von gesättigten und ungesättigten organischen Nitrilen zu den entsprechenden gesättigten Aminen.

Besonders geeignet ist der erfindungsgemäße Katalysator für die Herstellung von Alkylaminen und Alkylendiaminen durch Umsetzung von Alkannitrilen oder Alkandinitrilen mit 3 bis 18 Kohlenstoffatomen mit Wasserstoff in Gegenwart von Ammoniak. Besondere Bedeutung haben die erfindungsgemäßen Katalysatoren für die Herstellung von Hexamethylendiamin durch Umsetzen von Adipodinitril mit Wasserstoff in Gegenwart von Ammoniak erlangt. Hierbei hält man Temperaturen von 80 bis 140° C, vorzugsweise 110 bis 120° C und Drücke von 100 bis 400 bar, vorzugsweise 200 bis 300 bar ein. Die Hydrierung erfolgt vorteilhaft in Gegenwart von Ammoniak, das teilweise durch zurückgeführtes rohes Hydriergemisch, das im wesentlichen aus Hexamethylendiamin und Ammoniak besteht, ersetzt werden kann. Es hat sich bewährt, wenn das Volumenverhältnis von Adipodinitril zu Ammoniak wie 1: 2 bis 1: 20, vorzugsweise 1: 6 bis 1: 12 beträgt.

Der Gegenstand der Erfindung sei an folgenden Beispielen veranschaulicht.

## Beispiel 1

### Herstellung des Katalysators

600 kg nadeförmiger Lepidokrokit ($\gamma$-FeOOH) mit einem Chlorgehalt < 0,1 % und einem $Na_2O$-Gehalt < 0,1 %, hergestellt nach der DE-AS 10 61 760, mit einer spezifischen Oberfläche von 32 $m^2/g$, einer mittleren Nadellänge von 0,8 µm und einem Längen: Dicken-Verhältnis der Nadeln von 22: 1, einer Schüttdichte von 0,37 g/cm und mit einem pH-Wert von 7,2 werden in einem gerührten Festbett bei 400° C, 38 h mit 400 $Nm^3/h$ Wasserstoff zu metallischem Eisen (Fe > 95 %) reduziert (stöchiometrischer Wasserstoffüberschuß: 64). Anschließend wird in einem Stickstoffluftgemisch das pyrophore nadelige Metallpigment bei einer Temperatur von 60° C mit einer stabilisferenden Oxydschicht überzogen, wobei der Reduktionsgrad nicht unter 90 % absinken soll. Die Ausbeute beträgt 400 kg. Die Sättigungsmagnetisierung der Eisenteilchen beträgt 153 nT $m^3/g$ in einem 160 kA/M Meßfeld. Die Eisenteilchen haben eine spezifische Oberfläche von 7,2 m2/g (nach der BET-Methode) und besitzen in der elektronenmikroskopischen Aufnahme eine anisotrope geometrische Form (Nadel- bzw. Stäbchenform).

Zur Herstellung von geformten Massen mit Durchmesser 5 mm und einer Höhe von 4 mm wird das stabilisierte pulverförmige Metallpigment mit 2 Gew.% Graphit vermischt und unter Stickstoffabdeckung tablettiert. Die Druckhärte der Tabletten soll nicht unter 300 kp/cm² liegen.

## Beispiel 2

### Aktivierung und Verfestigung des Katalysators

In einem Reaktor der Länge 1800 mm und einem inneren Durchmesser von 160 mm werden 350 l der nach Beispiel 1 hergestellten Formlinge (Tabletten) eingefüllt und bei 360° C und 150 bar mit hohem Wasserstoffüberschuß (64-fach) über 24 h zum Zweck der Aufhebung der Passivierung behandelt. Der Wasserstoff wird dabei im Rreis über einen Kondensator zur Abscheidung des Reduktionswassers geführt. Der Reduktionsgrad beträgt > 98 %.

Unter diesen Aktivierungsbedingungen steigt die Druckhärte der Katalysatortabletten von 300 auf 800 bis 900 kg/cm².

Nach dem Abkühlen des Katalysators wird der Wasserstoff durch Stickstoff verdrängt. Mit einem maximal 0,5 Vol.% Sauerstoff enthaltendem Stickstoffluftgemisch wird der Katalysator erneut passiviert, wobei die Gasmenge so eingestellt wird, daß die Katalysatortemperatur nicht über 100° C steigt und der Reduktionsgrad des Katalysators nicht unter 90 % absinkt. Anschließend wird der Katalysator unter den oben genannten Bedingungen mit $H_2$ erneut aktiviert, wobei die Druckhärte der Tabletten weiter auf 1000 bis 1200 kp/cm² steigt. Der Reduktionsgrad beträgt > 98 %.

Durch Wiederholung von Passivierung und Reduktion kann die Druckhärte schließlich auf Werte bis 1500 kg/cm² gesteigert werden.

## Beispiel 3

### Hydrierung von Adipinsäuredinitril

Dem unter Beispiel 2 beschriebenen Reaktor, gefüllt mit 350 l des nach Beispiel 1 hergestellten und nach Beispiel 2 verfestigten und aktivierten Katalysators, werden in Rieselfahrweise bei 270 bar Wasserstoffdruck stündlich 85 l Adipinsäuredinitril und 510 l flüssigen Ammoniaks unter Kreisgasfahrweise (400 $Nm^3/h$) zugeführt. Die Temperatur des Zulaufgemischs beträgt 78° C und die des Reaktorausgangs liegt bei 110° C; das T max des Temperaturprofils liegt bei 119° C.

Die gaschromatographische Analyse des rohen Hexamethylendiamins nach Verdampfen des Ammoniaks aus dem Hydriergemisch ergibt 0,02 Gew.% Hexylamin, 0,09 Gew.% Azacycloheptan, 0,11 Gew.% 1,2-Diaminocyclohexan und 99,78 Gew.% Hexamethylendiamin sowie einen Aminocapronitrilgehalt $\leq$ 0,01 %. Der vorwiegend aus Bishexamethylentriamin bestehende Destillationsrückstand liegt bei 0,36 %. Es errechnet sich eine Selektivität von 99,4 % Hexamethylendiamin. Der Katalysator hatte nach einer Laufzeit von 700 Tagen unveränderte Aktivität und Selektivität ohne jegliche Regenerierung.

Die Zerfallsrate des Katalysators lag unter 0,5 %. Die Zerfallsrate ist der Anteil des Katalysators der ein Sieb mit einer Maschenweite von 3 mm passiert.

## Vergleichsbeispiel

γ-FeOOH wird nach Zusatz von 2 % Graphit zu Tabletten (Durchmesser 5 mm, Höhe 4 mm) verpreßt. Die Druckhärte wird auf 300 kp/cm$^2$ eingestellt. Danach werden die Tabletten mit Wasserstoff bei 360°C auf einen Reduktionsgrad von $\geq$ 95 % gebracht. Durch die Reduktion des γ-FeOOH sinkt die Druckhärte der Tabletten drastisch auf Werte $\leq$ 25 kp/cm$^2$. Die Tabletten zerfallen bei Berührung der zylindrischen Mantelfläche leicht in Scheiben.

Im Unterschied zu Tabletten, die aus bereits reduziertem γ-FeOOH hergestellt wurden, kann die mechanische Stabilität dieser Tabletten durch wiederholtes Passivieren und Reduzieren nicht verbessert werden.

## Patentansprüche

1. Geformte Eisenkatalysatormasse, enthaltend metallische Eisenteilchen, die aus Eisenoxidteilchen durch Kontakt mit Wasserstoff bei einer Temperatur von $\leq$ 500°C erhalten worden sind und einem Gleitmittel, gekennzeichnet durch eine Druckhärte von 850 bis 1500 kp/cm$^2$.

2. Katalysatormassen nach Anspruch 1, dadurch gekennzeichnet, daß sie 1 bis 5 Gew.% Gleitmittel enthalten.

3. Katalystormassen nach den Ansprüchen 1 und 2, dadurch gekennzeichnet, daß sie Graphit als Gleitmittel enthalten.

4. Katalysatormassen nach den Ansprüchen 1 bis 3, dadurch gekennzeichnet, daß man von anisometrischem gamma-Eisen(III)oxidhydrat ausgeht.

5. Verfahren zur Herstellung von geformten Eisenkatalysatormassen, enthaltend metallische Eisenteilchen und ein Gleitmittel durch

a) Reduktion von Eisenoxidteilchen mit Wasserstoff bei einer Temperatur von 250 bis 500°C zu metallischen Eisenteilchen

b) Passivieren der Eisenteilchen durch Behandeln mit einem molekularen Sauerstoff enthaltendem Inertgas

c) Pressen der passivierten Eisenteilchen mit Gleitmitteln zu Formkörpern

d) Aktivieren der Formkörper durch Behandeln mit Wasserstoff bei einer Temperatur von 250 bis 500°C,

dadurch gekennzeichnet, daß die aktivierten Formkörper mindestens noch einmal durch Behandeln mit molekularem Sauerstoff enthaltendem Inertgas passiviert und anschließend durch Behandeln mit Wasserstoff bei einer Temperatur von 250 bis 500°C aktiviert werden.

6. Verfahren nach Anspruch 5, dadurch gekennzeichnet, daß man die Passivierung und Aktivierung der Formkörper 1- bis 5-mal durchführt.

7. Verfahren nach den Ansprüchen 5 und 6, dadurch gekennzeichnet, daß man die anisometrischen Eisenoxidteilchen bis zu einem Reduktionsgrad von größer 95 % reduziert.

8. Verfahren nach den Ansprüchen 5 bis 7, dadurch gekennzeichnet, daß man beim Passivieren durch Behandeln mit molekularem Sauerstoff enthaltendem Inertgas einen Reduktionsgrad von 80 % nicht unterschreitet.

9. Verwendung der geformten Eisenkatalysatormassen nach den Ansprüchen 1 bis 8 zur Hydrierung von organischen Nitrilen zu den entsprechenden Aminen.

## Claims

1. A molded iron catalyst material containing metallic iron particles, obtained from iron oxide particles by contact with hydrogen at $\leq$ 500°C, and a lubricant, characterized by an indentation hardness of from 850 to 1500 kp/cm$^2$.

2. A catalyst material as claimed in claim 1, containing from 1 to 5% by weight of a lubricant.

3. A catalyst material as claimed in claims 1 and 2, containing graphite as lubricant.

4. A catalyst material as claimed in claims 1 to 3, wherein the starting material used is anisometric gamma-iron (III) oxide hydroxide.

5. A process for the preparation of a molded iron catalyst material containing metallic iron particles and a lubricant, by:

a) reducing iron oxide particles with hydrogen at from 250 to 500°C to metallic iron particles,

b) passivating the iron particles by treatment with an inert gas containing molecular oxygen,

c) compression molding the passivated iron particles with a lubricant to give moldings, and

d) activating the moldings by treatment with hydrogen at from 250 to 500°C,

wherein the activated moldings are passivated by treatment with an inert gas containing molecular oxygen and subsequently activated by treatment with hydrogen at from 250 to 500°C, these steps being carried out once or several times.

6. A process as claimed in claim 5, wherein the passivation and activation of the moldings are carried out from one to five times.

7. A process as claimed in claims 5 and 6, wherein the anisometric iron oxide particles are reduced until the degree of reduction is > 95%.

8. A process as claimed in claims 5 to 7, wherein, in the passivation by treatment with an inert gas containing molecular oxygen, the degree of reduction does not fall below 80%.

9. The use of a molded iron catalyst material according to claims 1 to 8 for the hydrogenation of organic nitriles to the corresponding amines.

**Revendication**

1. Matière catalytique façonnée à base de fer, comprenant des particules de fer métallique, obtenues par contact de particules d'oxyde de fer avec l'hydrogène a une température égale ou supérieure à 500°C, et un lubrifiant, caractérisée par une résistance aux pressions de 850 à 1500 kp/cm².

2. Matières catalytiques suivant la revendication 1, caractérisées en ce que la proportion du lubrifiant est comprise entre 1 et 5 % en poids.

3. Matières catalytiques suivant les revendications 1 et 2, caractérisées en ce que le lubrifiant est du graphite.

4. Matières catalytiques suivant les revendications 1 à 3, caractérisées en ce que le produit de départ est de l'hydrate d'oxyde ferrique γ anisométrique.

5. Procédé de préparation de matières catalytiques façonnées à base de fer, contenant des particules de fer métallique et un lubrifiant, par

a) réduction de particules d'oxyde de fer par l'hydrogène entre 250 et 500°C en particules de fer métallique,

b) passivation des particules de fer par traitement avec un gaz inerte contenant de l'oxygène moléculaire,

c) compression des particules de fer passivées avec un lubrifiant en éléments façonnés,

d) activation des éléments façonnés par un traitement par l'hydrogène entre 250 et 500°C, caractérisé en ce que les éléments façonnés activés sont soumis à au moins une passivation par le traitement avec du gaz inerte contenant de l'oxygène moléculaire, puis à une nouvelle activation par le traitement par l'hydrogène entre 250 et 500°C.

6. Procédé suivant la revendication 5, caractérisé en ce que la passivation et l'activation des éléments façonnés sont répétées une à cinq fois.

7. Procédé suivant les revendications 5 et 6, caractérisé en ce que les particules d'oxyde de fer anisométrique sont réduites jusqu'à un degré de réduction supérieur à 95 %.

8. Procédé suivant les revendications 5 à 7, caractérisé en ce que, lors de la passivation par traitement avec un gaz inerte contenant de l'oxygène moléculaire, l'on ne dépasse pas un degré de réduction de 80 %.

9. Utilisation de matières catalytiques façonnées à base de fer suivant les revendications 1 à 8 pour l' hydrogénation de nitriles organiques en amines correspondantes.